# EUROPEAN PATENT SPECIFICATION

(11) **EP 1 377 205 B1**
(45) Date of publication and mention of the grant of the patent: **29.07.2009**
(21) Application number: 02714240.5
(22) Date of filing: 10.04.2002
(51) Int. Cl.: A61B 5/00, H04R 3/12

(54) **PERSONALIZED INFORMATION DISTRIBUTION SYSTEM**
PERSONALISIERTES INFORMATIONSVERTEILUNGSSYSTEM
SYSTEME DE DISTRIBUTION D'INFORMATIONS PERSONNALISEES

(30) Priority: 11.04.2001 FI 20010767
(43) Date of publication of application: 07.01.2004
(73) Proprietor: Audio Riders Oy, 01800 Klaukkala (FI)
(72) Inventor: LAITINEN, Arvo, FIN-01900 Nurmijärvi (FI); PETSALO, Jyrki, FIN-02600 Espoo (FI); VUORI, Jarkko, FIN-40600 Jyväskylä (FI)
(74) Representative: Savela, Antti-Jussi Tapani
(86) International application number: PCT/FI2002/000300
(87) International publication number: WO 2002/082985

(56) References cited:
- EP-A2- 1 117 085
- US-A- 4 591 729
- US-A- 5 576 685
- US-A- 5 617 112
- US-A- 6 091 826

## Description

### Field of the Invention

The invention relates generally to intelligent information distribution systems, particularly to audio systems in which audio messages are given to users. In particular, the invention relates to information systems where an individual person or at most a small group of persons is simultaneously within the coverage of the information source.

### Background of the Invention

So-called intelligent sound reproduction systems in which the content of the information to be transmitted or the quality of the sound can be changed in accordance with environmental factors, for example, are intended for large, usually public spaces, where each loudspeaker unit is located in a different part of the space, such as a shop, shopping center, etc.

U.S. Patent 5,576,685 (Saito) discloses a system where loudspeakers located in a public space are controlled on the basis of the conditions prevailing in the space. The objective is to improve systems which always reproduce a certain piece of music with the same volume, tone, and tempo. For this, the system includes at least one sensor which can measure background noise, temperature, brightness or another corresponding feature and which controls, based on the measurement, the sound sequence transmitted by the loudspeakers, and the characteristics of the sound sequence, such as its volume.

U.S. Patent 6,091,826 (Laitinen et al.) in turn discloses a sound reproduction system for large spaces, in which the presence of people or their behavior, for example, can be monitored, in addition to the environmental conditions at each loudspeaker. The detection of the presence can be combined with detection of the identity of the person. Based on the monitoring, information dependent on the result of the monitoring is supplied from the loudspeaker. In this way individually targeted information, such as instructions or commercials, can be transmitted to different parts of the system, on the basis of how the listeners are located in the space, how they move in the space, and/or who they are.

On the other hand, small personal sound reproduction systems are passive and only obey the instructions given to them through a keypad. Examples of these are different portable players, such as MP3 players, or traditional radio receivers.

U.S. Patent 6,091,826 discloses proximity detectors, such as radars, which inform the server of the movements of people taking place in their environments. Then, in response to the movements, the server presents a program stored in a memory, the program including audio or visual information to be provided to the people. Also the presentation of the program can be controlled.

A drawback of the above known sound reproduction systems is that systems capable of adapting to the listener in real time cannot be implemented by them. This is because in these systems the state or ability of the listener cannot be taken into account; the listener affects the system only through his or her active choices and/or through predetermined parameters. Two persons approaching a loudspeaker in a similar manner thus cause a similar response (audio message), even though their current states and abilities for receiving the said audio message might be completely different.

U.S. Patent 4,591,729 discloses a control unit for a teaching device. The control unit switches the teaching device on when the test person is in so-called alpha-state, in which better learning ability and perception exist. V arious physiological signals, such as pulse, skin resistance, blood pressure, or respiration rhythm, may be converted to an electrical signal that is further applied to a detector. When the detector has found that the test person has reached the alpha-state, it turns the teaching device on whereupon the teaching device starts to convey learning information.

A drawback of the control unit of this patent is that the information content, in this case learning information, is fixed and left intact during the teaching session. The manner in which the information is presented to the user is independent of the current state of the user. Only the starting instant of the learning lesson is dependent on the state of the test person.

The main objective of the invention is to accomplish an intelligent information distribution system which can adapt to the personal and real-time ability of the user as well as possible and on that basis dynamically adjust the information flow and the manner it is presented so that they are as appropriate as possible for the recipient.

### Summary of the Invention

An objective of the invention is to devise an information distribution system which is suitable for personal use and can adapt to the current state of the user as well as possible, depending on the current cognitive, physical, mental and/or social performance of the user.

This objective is achieved with the solution defined in the independent claim 1.

The idea of the invention is to connect the user so that the user becomes a part of the system and his or her current state and abilities affect the information content and the manner in which the information is supplied to him/her. Thus the user is part of the system and of the control circuit affecting the information stream. The system of the invention includes sensors through which signals are obtained from the user, signals describing various somatic phenomena directly associated with the user. The phenomena are biophysiological. How the information is supplied to the user, how the supply and possibly other parameters are adjusted during the process, is determined on the basis of these signals. In this way the information flow is made as appropriate as possible for the user. In other words, when the state of the user is better known, the message given to him or her can be made more effective than before.

In a preferred embodiment of the invention, additional environmental monitoring supporting the actual measurement is performed in order to be able to evaluate the effect of the environmental conditions on the measurement signals. Additional measurements of this kind, which support the decision-making, can include measurements of the environmental temperature or air humidity, for example.

A further objective of the invention is to make the system suitable for different kinds of environments. For this purpose, another preferred embodiment of the invention includes regulation means for regulating the state of the environment (the presentation space and conditions) when necessary in order to render the response of the message as efficient as possible. Such control means can be included in the system even though it does not include the above-mentioned means for monitoring the environment. However, the system preferably includes both monitoring and regulating means. Different system alternatives are described in more detail below.

The system can be used for many different purposes, either as such or combined with other systems. Applications to be mentioned here are research (for example, medical), education, rehabilitation, therapy, systems based on virtual reality in which a tailored audio space is part of the system, or entertainment, such as games played via a network. The system can also be used together with other systems, for example, for selecting the most suitable channels from a large group of radio and TV channels. The system can also form an ergonomic man-machine system.

The core of the system can comprise a sound reproduction device like an MP3 player, and thus the system can form a future evolution version of the MP3 player.

Depending on the nature of the system, the control of the audio information can only take place locally, but the control can also be implemented as a higher-level control, in which case each local system is controlled individually based on the measurement data obtained from a plurality of distinct local systems.

### Brief Description of the Drawings

In the following, the invention and its preferred embodiments are described more closely with reference to the examples shown in FIG. 1 to 6 in the appended drawings, wherein:
FIG. 1 shows the general principle of the system of the invention in its basic embodiment,
FIG. 2 shows the general principle of the system of the invention in an enlarged embodiment,
FIG. 3 shows a more detailed implementation of a system of the invention,
FIG. 4 illustrates the use of the system of the invention in remote rehabilitation,
FIG. 5 illustrates a system utilizing a higher-level control, and
FIG. 6 illustrates the use of the system of FIG. 5 for rehabilitation of bed patients.

### Detailed Description of the Invention

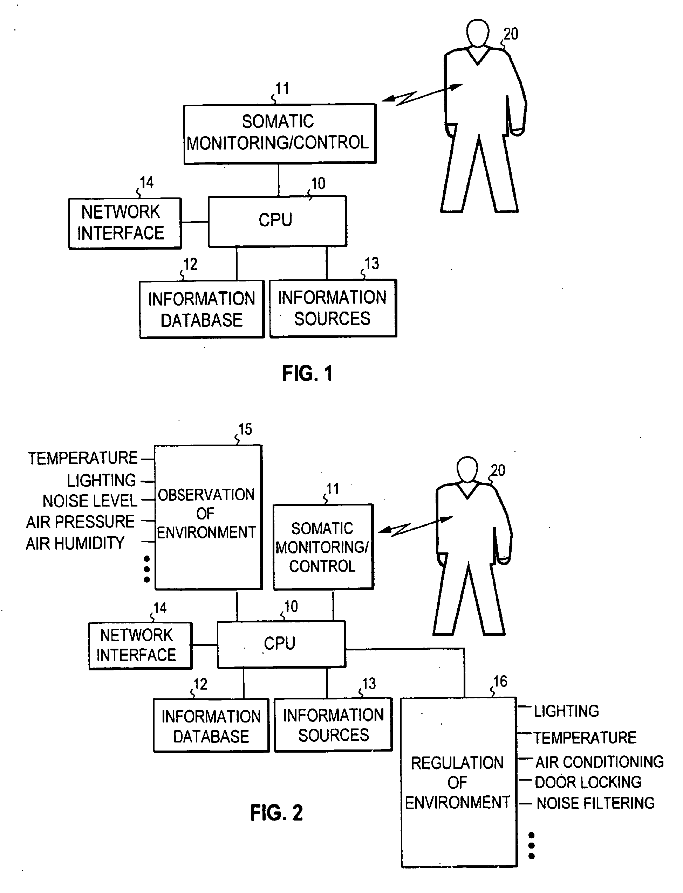

FIG. 1 illustrates the basic principle of the system according to the invention. The core of the system is formed by a central unit 10, which controls the operation of the system. A separate monitoring unit 11 measuring somatic phenomena associated with a user 20 is connected to the central unit. Based on these measurements, the central unit selects the information to be presented to the user and the manner the information is presented. The control of the manner of presentation refers generally to different ways of presenting the same content, and it can include the control of different presentation parameters, such as volume, tone, tempo, number of repetitions, etc., and the use of spatial sound, for example, so that the sound can be heard from the desired part of the listening space. The information available (e.g. the programs), from which the selection is made on the basis of the measurements, is stored in an information database 12, which is typically on a hard disk but it may also be in a component memory, for example. Alternatively, the programs can be downloaded through a network interface 14 from the network, or a wireless interface can be implemented for them as a WLAN/Bluetooth implementation in the 2.4 GHz license free band, for example. The information is presented through the information sources 13, which typically include at least a loudspeaker unit, as described below. The basic idea of the invention is to connect a human being as a part of the system by measuring a somatic/physiologic phenomenon from her/him and by selecting, based on the measurement, the content of the information to be presented and/or the manner of presentation so that it is as suitable as possible with respect to the current state of the user.

The idea behind the system according to the invention is that a human being subconsciously and consciously observes his or her environment and his or her state therein. The current state or capability of a human being consists of his or her physical, cognitive, mental, and social performance. These vary individually in accordance with time, health, and state of activeness, among other factors, and they can be independent of each other. Modem brain research (including new imaging techniques) has in turn proved that the sense of hearing also functions subconsciously outside the active will of the person. When walking down a street, for example, a person can recognize his or her name as part of the sound environment. The long memory trace of sound and a strong coupling to emotion and imagination are a huge resource as such. The behavior and performance of an individual can be significantly affected by sound messages. By means of the system according to the invention, the behavior of a person and his or her current physical, mental, cognitive, or social performance, or all these factors, can be evaluated by measuring one or more parameters correlating with at least one such factor. Instead of, or in addition to, the measurement of absolute values, the relative state of an individual as compared to himself/herself or to a certain reference can be evaluated.

Thus one or more somatic phenomena correlating in the above-described manner with one of the said performances can be monitored by the monitoring unit 11. These phenomena can be divided into biophysical phenomena, which can indicate events at the tissue or cell level, and into phenomena associated with behavior or the nature of movements described by psychomotor quantities. Different alternatives for the measurements are listed in the following. Already today many of these measurements can be performed non-invasively and wirelessly.

The following are examples of biophysiological phenomena to be monitored.

EMG (Electromyography) measures the electric activity caused by muscular contractions. This measurement can indicate tension of muscles, fatigue, or physical work (movement), for example. EMG can be used, for example, in rehabilitation by presenting a rehabilitation program for the patient corresponding to his or her current condition. An EMG measurement is also suitable for management of stress and pain, for example, whereby the (audio)information and its presentation parameters are selected according to the current stress or pain status of the user.

An EKG measurement can be used mainly for monitoring the pulse. The pulse reflects the physical performance and/or the degree of physical load, state of activity, or tension, for example.

EEG (ElektroEnkefaloGrafia), i.e. brain wave registration, can be used for evaluation of the state of activity or power of concentration, for example, whereby it can be used as an aid in education to select ideal content/duration/repetition for the study session.

Electric conductivity of the skin can be used for evaluation of stress or psychic tension, for example.

Temperature measurement in a desired part of the body can also be used to evaluate stress, tension, muscle motoric work, or health, for example. Temperature can be measured non-invasively by an infrared heat camera, for example. In this way also the temperature differences in various parts of the body can be monitored simultaneously.

Measurements relating to respiration (e.g. rhythm) can in turn correlate with physical performance or strain, for example.

Blood pressure can also reflect strain, at least if other information, such as the normal blood pressure of the user, is available.

Similar to measurements of tissue or cell level, also measurements of hormone levels and microbiological quantities can be performed. The system can include micro-sensors, which control the supply of a measurement substance to the human system, the response in the system being measured dynamically. Thus somatic monitoring can extend to a control which in some way concerns the somatic operation of the user. On the one hand, the control can support measurement, for example, measurement of certain factors can require the supply of a measurement substance etc. in order for the measurement to be performed. On the other hand, the control may be needed in order to move the person to the operation range of the control loop of the system.

As mentioned above, also parameters directly related to the physical behavior, such as the eye movements or other movements of the user. The former can indicate attentiveness and the latter haste, for example. Both can be measured by a video camera, for example, and the movements of the user can also be measured by acceleration transducers or a gyroscope, for example.

Speech recognition can be used for evaluation of tension and social activity. In addition, less sophisticated equipment can be used to evaluate certain parameters from speech without recognition, such as pitch, number of pauses, speed of speech, which can adequately characterize the psychophysical state of the speaker.

It is to be noted that the above-described measurements do not necessarily correspond to measurements made in a laboratory, in which absolute values are obtained, but it is enough if changes relating to the same user can be solved by the measurements. Thus, the user himself/herself acts as a reference, and only a change is of significance.

Movement information obtained from the user (from his/her eyes, for example) can be used, for example, in museums or corresponding exhibition spaces for supplying suitable information according to the movements of the user. When the user moves with determination (according to an acceleration transducer, for example), the information can be more general and can change automatically according to the location (the location can be concluded, by means of a DGPS receiver or by integrating the data received from the acceleration transducer). Stops made by the user automatically increase the information content and the depth of the content. If the acceleration sensor is of a 3D- type, turning the eyes to the ceiling, for example, can direct a description of a ceiling fresco or the like to the earpieces. The nature of the movements of the user can thus be an indication of his or her state (for example wishes/intentions), the nature being described by signals obtained from equipment carried by the user himself/herself, by means of which equipment the user is connected as a part of the control loop.

The user can be connected to the system through a wireline connection or wirelessly. The sensors have traditionally been based on a wireline connection, but the proportion of wireless sensors will increase as wireless short-range communication becomes more common in all kinds of electronic equipment. The wireless sensors can utilize a short-range RF technique or a Bluetooth technique, for example. Biosensor technology will also develop quickly. For example, at the moment there are sensors on the market based on wireless escort memories, the sensors being readable from within a nearby distance. It has also been possible to attach a sensor part to these sensors so that temperature, pressure, etc. can be measured by them. The said sensors can be used for wireless measurement, for example, from the surface of the skin or even from within the skin as an implant.

The measurements on the user can also be made so that the sensor is not attached to the user, but a physiological quantity, such as pulse or respiration is measured from the mechanical movement of a bed or a chair, for example, by means of a foil which converts mechanical movement to an electrical signal.

FIG. 2 illustrates three other embodiments of the system in accordance with the invention. In addition to the units mentioned above, the system can namely include observation means 15 for monitoring the environment of the user, regulation means 16 for regulating the environmental conditions, or both the observation means and the regulation means (three embodiments).

The observation means 15 can be used to monitor desired parameters or phenomena relating to the environment where the user is. The quantities measured from the environment vary typically according to which quantities are measured directly from the user, in order to take into account the effect of the environmental conditions on the actual physiological measurement. For example, the temperature of the environment may be an important piece of information in order to be able to evaluate the state of the user by means of a body temperature measurement or a skin conductivity measurement on the user. Similarity, the brightness of the lighting can affect the EEG, for example, or air humidity can affect skin conductivity. Air pressure, in turn, can affect pulse and the frequency of respiration, for example.

By means of the regulation means 16 a quantity is controlled, whose control boosts the effect of the response of the information given to the user. As the quantity to be controlled can even be such that it does not relate to the somatic control but otherwise boosts the response, the regulation means can belong to the system even if there are no observation means 15 in the system. The locking of doors or lighting can be mentioned as an example of such, provided that a quantity is measured from the user which does not correlate with the intensity of the lighting.

The quantities to be measured from the environment are such that they indicate whether a control has to be performed by the regulation means in order to enhance the response, and if so, how the control has to be performed. An example of this is a measurement of the noise in the environment, as a result of which an active noise cancellation can be initiated based on an opposite-phase signal, for example.

It is yet to be noted that FIG. 2 discloses examples of quantities/items which can be controlled or monitored in the systems according to the invention. Thus the monitoring or control of all quantities/items does not have to be incorporated into the same system.

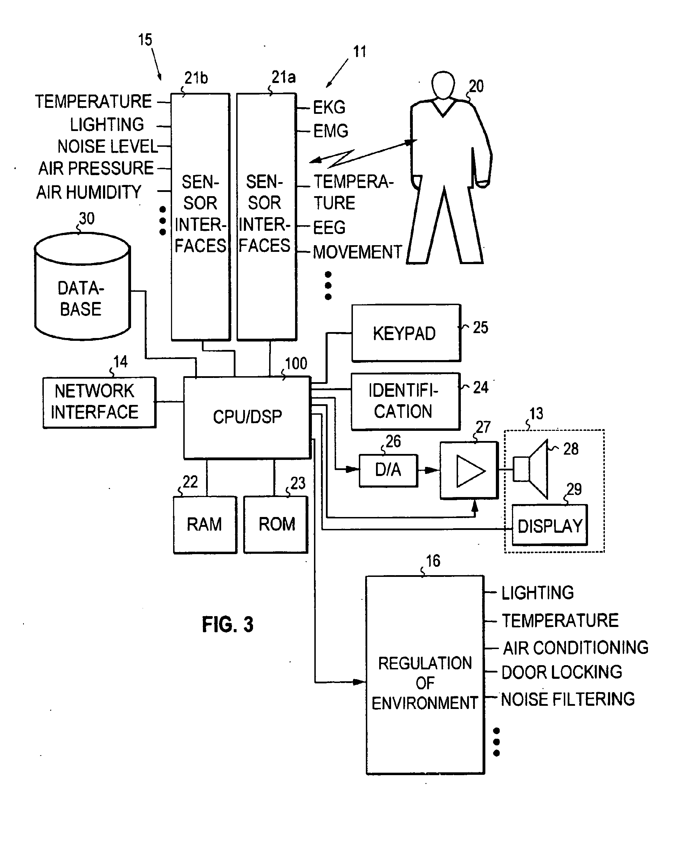

FIG. 3 shows a more detailed structure of one system in accordance with the invention. The core of the system is formed by a CPU or a signal processor unit 100, which is termed "a processor unit" below. Sensor interface units 21 a and 21 b are connected to the processor unit, the sensors being connected to the sensor interface units either via wireline connections or wirelessly. Sensor interface unit 21a is for the measurements made on the user, i.e. for somatic control, and sensor interface unit 21 b is for monitoring the environment, although all the sensors can be connected to the same interface unit.

Sensor interface unit 21a can also include a camera interface, if a camera is used for monitoring. A program memory 23 and a data memory 22 (RAM or DRAM) are associated with the processor unit, the program memory to store the software used by the processor and the data memory to store the data used by the processor. The program memory can in practice consist of so-called Flash memory cards. The software can also be downloaded or updated through a network interface. Efficient processing algorithms or fuzzy logic inference can be implemented by the processor unit. The information to be presented can thus be modified flexibly by the processor unit using, for example, different kinds of filters, and the environmental factors can be regulated in a desired way in order to enhance the response.

A database 30 can further be associated with the processor unit, the database storing intelligence for the apparatus, such as rules and measured responses of previous information deliveries, or control values according to which the selection of the programs and their presentation parameters occur.

The information source 13 includes typically a loudspeaker 28 whose task is to convert the electrical signal into an acoustic signal. The processor unit controls the loudspeaker through a D/A converter 26 and an amplifier unit 27. In addition to the loudspeaker, the information source can also include a display unit 29, for example, for displaying graphic or textual information.

The apparatus preferably also includes an identification device 24, which identifies the user of the apparatus. The identification can take place using any known technique, for example, a smart card and a card reader. Each user then has a smart card of his or her own, which stores user-specific information. Using this information, the program collection belonging to the application associated with the user, and possibly other information relating to the application, such as control information, can be retrieved through the network, for example. Different users can in this way utilize the system for different purposes.

The apparatus can further include a keypad 25 which can be used to control the system and to measure the reaction time of the user, for example, if it is needed for the evaluation of the state of the user.

Audio information downloaded from the network can be stored into the mass memory 30. Depending on the application, this information can be in the form of ready-made programs or as logic audio sequences, of which each can form one record, for example. The message contained by a logic audio sequence includes one logic entity. The records to be stored can be formed so that certain words, sentences, or parts of sentences are stored only once, and the processor combines the records sequentially so that they form the desired message.

Through a system provided with regulation means 16, it is possible to implement ergonomic man-machine systems for different purposes. As an example can be mentioned the incorporation of the system into a car, for monitoring the fatigue of the driver and for giving audio warnings. These audio warnings can be enhanced, for example, by means of regulation means which open the window or the radio, cause vibration of the seat, etc. The regulation means can also be incorporated into intelligent clothing, for example, which is provided with a massage function for enhancing the response of the audio message. In man-machine systems like these, the information can also be delivered, in addition to loudspeakers, through glasses, which are put on, or through micro displays, whereby the processor can modify the video information to be presented similarly to the audio information.

The regulation means can also include different kinds of mechanisms whereby the desired conditions or a desired (imaginary) situation are simulated in order to enhance the response. These can be, for example, mechanisms whereby desired weather, climate, or movement conditions, or scents corresponding to the desired situation, for example, are produced in the space where the information is delivered.

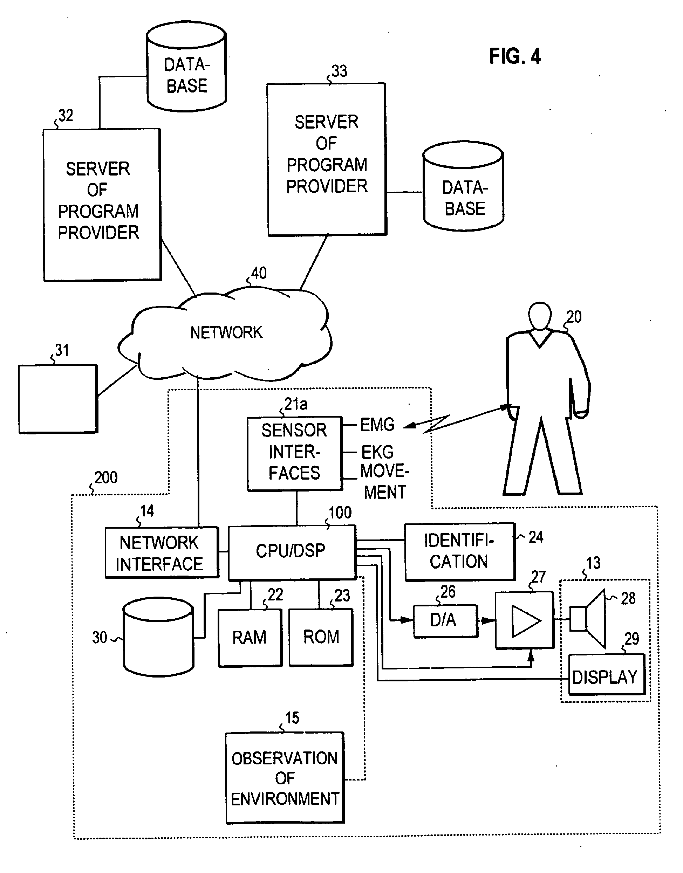

FIG. 4 shows an example of one system in accordance with the invention, the system being in this example used for personal rehabilitation through a communication network. In the rehabilitation, EMG and EKG signals and movement can be measured of the user, for example. Several different content providers can be in the network 40 which produce programs for one or more applications. Let us assume that there are at least two content providers for the rehabilitation, the servers being denoted with reference numerals 32 and 33. The rehabilitator or other expert rehabilitating the user first downloads at his or her own server or terminal 31 suitable start programs and initial parameters from the database of one or more service providers to the mass memory, such as the hard disk of the system of the user. Alternatively, the rehabilitator can store a software profile in the smart card of the user, whereby the process unit downloads the said programs after it has verified the identity of the user with the identification device 24.

During the rehabilitation event the above-described measurements (and possibly also monitoring of the environment) are performed and the rehabilitation instructions given through the loudspeaker 28 are controlled, e.g. their content, level of difficulty, duration, and/or number of repetitions.

Control instructions can be downloaded to the apparatus or smart card along with the start programs, the control being performed in accordance with the instructions on the basis of varying measurement results.

In accordance with the progress of the user, new programs are downloaded to the apparatus. The rehabilitation staff can also allow the user to access the program data of different service providers as the rehabilitation proceeds. For this purpose the rehabilitation staff can retrieve measurement data for its terminal or server from the user equipment in order to evaluate the progress of the person and to give new instructions. The rehabilitation staff can also (automatically) get reports or warning-like information of different events from the system.

The same equipment can also be used for different applications and thus the apparatus can be used for some other purpose after the rehabilitation session, for example, for entertainment, whereby also the objectives (and the measurements) change automatically.

In an individual local system 200 the use of sound information is analyzed and optimized locally. At the local level the operation is developed and maintained for activating the customers. At the local level it is also observed most quickly, what type of activities (games, etc.) are the best ones for obtaining the best result. Moreover, the local level generally offers the best point of view of in which direction the activities should be developed in order to improve the results. The RAI index, for example, can be used as a meter for the activity of the customers, in which case a clear scalar quantity is obtained for the activity and ability.

Although the local level generally offers the best point of view of how the existing services should be developed, creating completely new activities at the local level is generally difficult. This is why the system can in some cases include a higher-level control, by means of which methods found to be good at the local level can be analyzed and combined. In this case dependencies "action →improvement of RAI index" can be obtained from all local systems.

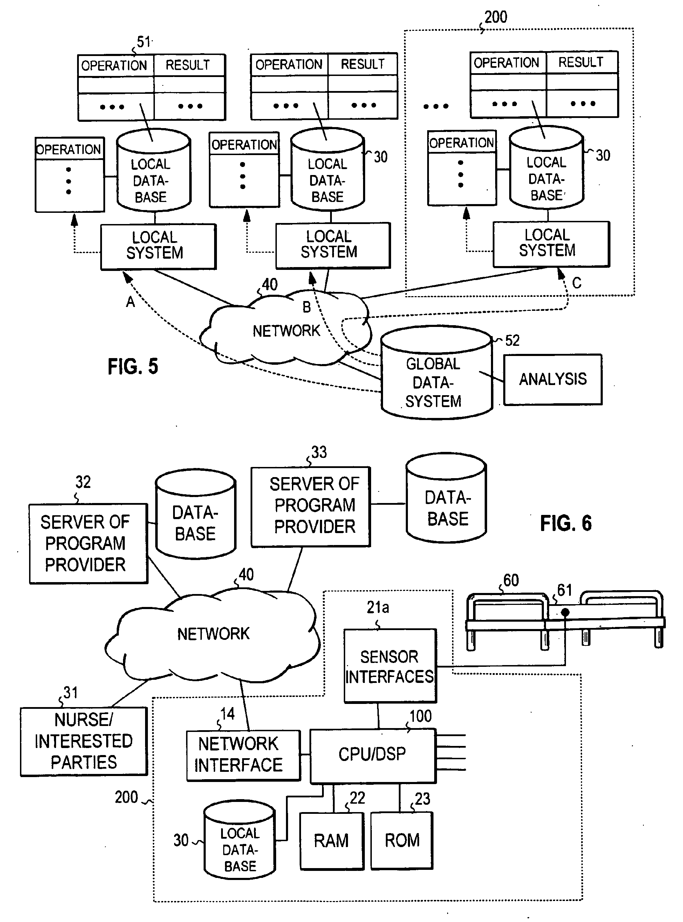

In order for reasonable operation models to be beneficial also at other local sites, there must be a connection between them. This connection is the higher-level control implemented through network 40, one or more servers in the network 40 participating in the control. The objective of the control is to combine the causal connections detected in the local systems 200 and to control the local systems on the basis of this combined information. FIG. 5 illustrates this principle. A relation database 51, for example, is created in the system at each local level, the database to comprise certain operations performed at the local level and the locally measured results to correspond to the operations. These results are transferred through the network 40 to a higher-level system 52, where the desired analysis is performed for the operation/RAI-index pairs in question. The local systems are controlled on the basis of the analysis results (arrows A, B, and C). The response of the sound programs can thus be seen as changes in the resulting parameters of the quantities measured from the people. The results are collected to the higher-level system for analysis.

For example, sound information can be classified in different categories on the basis of the information content. There should be as many of these categories as possible, and it does not matter if these overlap. Also the activation operations of the customers are classified, in respect to the number of interactions, sound information classes and/or question/answer pairs, for example. The number of activation operation classes is not restricted either; generally the more classifications there are, the better it is.

The higher-level control system can be implemented in many different ways. In the control, the following items can be utilized, for example:
- statistical analysis,
- multiple variable control,
- neural computation, e.g. self-organizing maps.

The use of a self-organizing map is preferable in the sense that not much knowledge about the process to be controlled is required. The number of parameters and their interdependencies are completely open. In the high-level control a self-organizing map (SOM) is constructed of the classes of sound information and operations and of their effect on the results measured from the customers. The parameters of the activation operation classes form an N dimensional map, the effects of the operation (which are positive or negative) being input as a point on the map. In this way the components whose effect is generally strong can be selected from the operations found to be good at the local level. In addition, high-level control combinations of operations ascertained as good at the lower level can be found by means of the self-organizing map, the combinations being better than the operations separately. In this way those acting locally can be directed to take more into consideration operations used at other sites. The self-organizing map efficiently finds statistical dependencies between different operations.

The operation classes can be as follows, for example:
query/answer type game
the questions include current information
the number of activation events is 20 in an hour
the number of activation events is 40 in an hour
music at least every 10 minutes.

These classifications are in use in all local systems. When a new operation program is created, it is classified according to the classes in use. If necessary, new classes can be created, whereby these new classes are immediately communicated to the other sites.

Let us assume that in one system a new query/answer type game has been developed. It has been classified and its effect on the RAI index has been analyzed in the local system: it has increased the average activity index by +3. The self-organizing map of the global data system 52 is informed of this information. In another local system it has been discovered that it is beneficial to play music to customers every 10 minutes. In this case the music has increased the activity index by +1. Now a denser area is formed for these classes on the map. It can be seen that the above operations effectively increase the index. Information about this can be supplied to the local level, whereby the activating possibility of a certain type of music is noticed in one local system and the beneficial effect of the query/answer type game in another. Thus, by means of the high-level control both systems can leam from the results of other local systems.

FIG. 6 illustrates the use of a system according to FIG. 4 and 5 for rehabilitation of bed patients. In this case the system can be based only on the local control; however, it is preferably a system according to FIG. 5, which is based on a higher-level control, whereby the global data system comprises server 33 of a service provider, the self-learning system of the above kind, for example, being created in the server on the basis of the measurement data collected from the local systems 200.

The rehabilitator can construct at his or her terminal 31 a modular rehabilitation program for the person or group on the basis of the knowledge obtained from the local data system.

In the example of FIG. 6 a person is placed in bed 60, which is provided with sensors. These can be, for example, located in the sensor mattress 61 in the bed of the person to be rehabilitated, similarly to the description in the WO 98/34540 publication, for example. In this way factors such as movements, pulse, and respiration can be measured as a function of time, as well as changes in the weight of the person.

The said parameters can be used, for example, in the physical rehabilitation of persons who have been in long-term bed care. By measuring movement (and pulse and respiration) variables from different sectors of the mattress, the sound-implemented rehabilitation instruction can be adjusted as controlled by the measurement parameters. For example, the agility and response of a person in turning exercises and the muscular shape (the response as a force) can be set to control the presentation speed, duration and repetitions of the physical exercises. Prior development and background skills can be obtained from local database 30, when the identification of the person has been made by means of the escort memory in the wristband, for example.

The presentation information, the environmental parameters, and the responses measured from the person are transferred to the global data system (32 or 33) from which the values of the presentation parameters are obtained. The presentation of the programs can occur either on-line or offline.

In an application of the above kind a possibility can also be given for interested parties involved with the person in care to follow the results of the rehabilitation or sleep and its parameters, for example, over the network either from a local database 30 or from a global database 52.

Instead of a bed, a chair can also be used in geriatry, for example, whereby the chair includes mattress sensor elements in locations such as the hand grips, the back rest, and/or the lumbar region. These elements act in the above-described manner as an information collecting unit which collects data for the local data system. A loudspeaker or a similar information source can be integrated with the chair, for example, with the raised back rest of the chair, the information content being presented through the information source. In other respects a system provided for a chair can be as described above.

A local system 200 according to the invention can be implemented, for example, in one portable player which is preferably based on a SOC solution (System-On-Chip), such as the current MP3 player.

Although the invention was described above with reference to the examples shown in the appended drawings, it is obvious that he invention is not limited to these, but may be modified within the definition of the invention given in the appended patent claims. For example, the system can be combined with other systems, such as systems where the information to be presented is ready. As an example can be mentioned the reception of (digital) radio and television transmissions, whereby the system can suggest, having made measurements of the above kind, the most suitable channels from the dozens or hundreds of channels available, in accordance with the current state of the user. The information source of the system can thus be a part of another system. The system can also be a part of the known audio systems described in the beginning, which are intended for large spaces. Also the quantities to be controlled, such as content, volume of sound, tone, tempo, reverberation, repetition of the same content, etc. depend on the application used. The use of spatial sound, so that the sound can be heard from the desired part of the listening space, can be included in the quantities to be controlled. The system can also be connected to external systems, such as building automation systems, whereby intelligent building automation for regulating the temperature, lighting, locking of doors, access control etc. is incorporated into the system, in which case no extra means are needed for observation and regulation of the environment. When combined with building automation, the system can report information concerning the house or room by sound and give instructions to the user or via the network also to other parties (for example the so-called call center service). A small group can also use the system according to the invention at the same time, especially if the group is homogenous enough. In this case the selections can be made on the basis of the averages of results obtained from the users, or utilizing the so-called fuzzy logic.

## Claims

1. Information distribution system, comprising
- at least one presentation device (13) for presenting information to a user,
- control means (10, 100, 52) for controlling the operation of said at least one presentation device (13), and
- monitoring means (11) for obtaining measurements, the control means being responsive to the monitoring means (11) for controlling the operation of the presentation device on the basis of the measurements, the monitoring means including measurement means for measuring biophysiological phenomena directly from the user,
wherein the control means controls the operation of the presentation device on the basis of the biophysiological measurements being performed by the measurement means and the control means (10, 100, 52) selects the program to be presented to the user on the basis of the biophysiological measurements, the program to be selected being stored in an information database (12) which contains a plurality of programs, and wherein the manner of the presentation of the program is dependent on the results of the biophysiological measurements.

2. A system according to claim 1, **characterized in that** the measurement means are adapted to make at least one type of measurement from a group including measurements of the EMG, EEG, and EKG, and measurements indicating skin conductivity, temperature, blood pressure and frequency of respiration.

3. A system according to claim 1, **characterized in that** the monitoring means further include observation means (15) for measuring the state of the environment of the user, whereby the control means are also responsive to the observation means.

4. A system according to claim 1 or 3, **characterized in that** it further includes regulation means (16) for regulating the state of the environment of the user, whereby the control means are adapted to control the regulation means.

5. A system according to claim 1, **characterized in that** the measurement means comprise sensors located on the user, the sensors being adapted to measure the nature of movements of the user.

6. A system according to claim 1- or 5, **characterized in that** the presentation device is incorporated into a portable player.

7. A system according to claim 1, **characterized in that** the control means comprise
- local control means (10, 100), operatively connected to control the presentation device, and
- global control means (52), operatively connected to control the local control means via a communication network (40).

8. A system according to claim 7, **characterized in that** the global control means are adapted to control several different local control means which are separate from each other.

## Patentansprüche

1. Informationsvertriebssystem, bestehend aus
- mindestens einem Präsentationsgerät (13) zur Darstellung von Informationen für einen Benutzer,
- Steuervorrichtungen (10, 100, 52) zur Steuerung des Betriebs von genanntem, mindestens einem Präsentationsgerät (13), und
- Überwachungsvorrichtungen (11) zum Erhalten von Messungen,
wobei die Steuervorrichtungen auf die Überwachungsvorrichtungen (11) regieren müssen, um den Betrieb des Präsentationsgerätes basierend auf den Messungen zu steuern, wobei die Überwachungsvorrichtungen auch Messvorrichtungen zur Ermittlung von direkten biophysiologischen Phänomenen des Benutzers umfassen müssen,
wobei steuern die Steuervorrichtungen den Betrieb des Präsentationsgerätes basierend auf den von den Messvorrichtungen durchgeführten biophysiologischen Messungen und die Steuervorrichtungen (10, 100, 52) wählen das dem Benutzer zu präsentierende Programm basierend auf den biophysiologischen Messungen, das auszuwählende Programm ist in einer Informationsdatenbank (12) abgespeichert, in der eine Vielzahl von Programmen abgelegt ist deren Präsentation von den Ergebnissen der biophysiologischen Messungen abhängt.

2. System nach Anspruch 1, **dadurch gekennzeichnet, dass** die Messvorrichtungen so angepasst sind, dass diese mindestens einen Messtyp aus einer Gruppe von Messungen, darunter EMG, EEG und EKG durchführen, sowie Messungen der Hautleitfähigkeit, Temperatur des Blutdrucks und der Atemfrequenz.

3. System nach Anspruch 1, **dadurch gekennzeichnet, dass** die Überwachungsvorrichtungen weitere Beobachtungsvorrichtungen (15) zur Messung des Umgebungszustands des Benutzers beinhalten, wobei die Steuervorrichtungen auch auf die Beobachtungsvorrichtungen reagieren.

4. System nach Anspruch 1 oder 3, **dadurch gekennzeichnet, dass** es weitere Regulierungsvorrichtungen (16) zur Regulierung des Umgebungszustands des Benutzers beinhaltet, wobei die Steuervorrichtungen so angepasst sind, dass sie die Regulierungsvorrichtungen steuern können.

5. System nach Anspruch 1, **dadurch gekennzeichnet, dass** sich die Messvorrichtungen auf am Benutzer befindlichen Sensoren basieren, die wiederum an die Messung der Bewegungen des Benutzers angepasst sind.

6. System nach Anspruch 1 oder 5, **dadurch gekennzeichnet, dass** das Präsentationsgerät in ein tragbares Wiedergabegerät integriert ist.

7. System nach Anspruch 1, **dadurch gekennzeichnet, dass** sich die Steuervorrichtungen zusammensetzen aus
- lokalen Steuervorrichtungen (10, 100), die operativ mit der Steuerung des Präsentationsgerätes verbunden sind, und
- globale Steuervorrichtungen (52), die operativ mit der Steuerung der lokalen Steuervorrichtungen über ein Kommunikationsnetzwerk (40) verbunden sind.

8. System nach Anspruch 7, **dadurch gekennzeichnet, dass** die globalen Steuervorrichtungen an die Steuerung unterschiedlicher lokaler Steuervorrichtungen angepasst sind, die voneinander getrennt sind.

## Revendications

1. Un système de distribution de l'information, comprenant
- au moins un dispositif de présentation (13) pour présenter des informations à un utilisateur,
- des moyens de contrôle (10, 100, 52) pour contrôler ledit fonctionnement d'au moins un dispositif de présentation (13), et
- des moyens de surveillance (11) pour obtenir des mesures, les moyens de contrôle répondant aux moyens de surveillance (11) pour contrôler le fonctionnement du dispositif de présentation sur la base des mesures, les moyens de surveillance incluant des moyens de mesure pour mesurer des phénomènes biophysiologiques directement de l'utilisateur,
dans lequel les moyens de contrôle contrôlent le fonctionnement du dispositif de présentation sur la base des mesures biophysiologiques étant exécutées par les moyens de mesure et les moyens de contrôle (10, 100, 52) sélectionnent le programme à être présenté à l'utilisateur sur la base des mesures biophysiologiques, le programme à être sélectionné étant stocké dans une base de donnée d'informations (12) qui contient une diversité de programmes, et dans lequel la manière de présenter le programme dépend des résultats des mesures biophysiologiques.

2. Un système selon la réclamation 1, **caractérisé par** des moyens de mesure adaptés pour effectuer au moins un type de mesure à partir du groupe incluant les mesures de EMG, EEG, et EKG, et des mesures indiquant la conductivité de la peau, la température, la pression artérielle et la fréquence de respiration.

3. Un système selon la réclamation 1, **caractérisé par** des moyens supplémentaires de surveillance comprenant les moyens d'observation (15) pour mesurer l'état de l'environnement de l'utilisateur, par lequel les moyens de contrôle répondent également aux moyens d'observation.

4. Un système selon la réclamation 1 ou 3, **caractérisé en ce qu'**il inclut en plus des moyens de régulation (16) pour réguler l'état de l'environnement de l'utilisateur, par lequel les moyens de contrôle sont adaptés pour contrôler les moyens de régulation.

5. Un système selon la réclamation 1, **caractérisé par** des moyens de mesure comprenant des détecteurs situés sur l'utilisateur, les détecteurs étant adaptés pour mesurer la nature des mouvements de l'utilisateur.

6. Un système selon la réclamation 1 ou 5, **caractérisé par** l'incorporation du dispositif de présentation dans un lecteur portable.

7. Un système selon la réclamation 1, **caractérisé par** des moyens de contrôle comprenant des
- des moyens de contrôle locaux (10, 100), connectés de manière opérationnelle pour contrôler le dispositif de présentation, et
- des moyens de contrôle globaux (52), connectés de manière opérationnelle aux moyens de contrôle locaux via un réseau de communication (40).

8. Un système selon la réclamation 1, **caractérisé par** des moyens de contrôle globaux adaptés pour contrôler différents moyens de contrôle locaux séparés des uns des autres.
